# EUROPÄISCHE PATENTSCHRIFT

(11) **EP 2 591 132 B1**
(45) Veröffentlichungstag und Bekanntmachung des Hinweises auf die Patenterteilung: **30.12.2015**
(21) Anmeldenummer: 11802999.0
(22) Anmeldetag: 15.06.2011
(51) Int. Cl.: C22C 23/04, C22C 23/06, C22F 1/06

(54) **MAGNESIUMLEGIERUNG**
MAGNESIUM ALLOY
ALLIAGE DE MAGNÉSIUM

(30) Priorität: 06.07.2010 AT 11342010
(43) Veröffentlichungstag der Anmeldung: 15.05.2013
(73) Patentinhaber: AIT Austrian Institute of Technology GmbH, 1220 Wien (AT)
(72) Erfinder: SCHIFFL, Andreas, A-5280 Braunau (AT); MINGLER, Bernhard, A-1110 Wien (AT)
(74) Vertreter: Wirnsberger, Gernot
(86) Internationale Anmeldenummer: PCT/AT2011/050001
(87) Internationale Veröffentlichungsnummer: WO 2012/003522

(56) Entgegenhaltungen:
- EP-A1- 1 842 507
- YU ET AL: "Microstructure and mechanical properties of ZK60-Yb magnesium alloys", MATERIALS SCIENCE AND ENGINEERING A: STRUCTURAL MATERIALS:PROPERTIES, MICROSTRUCTURE & PROCESSING, LAUSANNE, CH, Bd. 478, Nr. 1-2, 28. Februar 2008 (2008-02-28), Seiten 101-107, XP022503446, ISSN: 0921-5093, DOI: 10.1016/J.MSEA.2007.09.027

## Beschreibung

Die Erfindung betrifft eine Magnesiumlegierung sowie deren Verwendung und ein Implantat aus einer Magnesiumlegierung.

Des Weiteren betrifft die Erfindung ein Verfahren zur Herstellung eines Körpers, insbesondere eines Implantats, aus einer Magnesiumlegierung.

In der Implantattechnologie werden gegenwärtig Implantate aus Magnesiumlegierungen intensiv als biodegradierbare medizinische Produkte, die im menschlichen oder tierischen Körper eingesetzt werden, untersucht. Hintergrund dabei ist, dass Implantate aus Magnesiumlegierungen, je nach Beschaffenheit der jeweiligen Legierung, durch Körperflüssigkeiten auflösbar sind. Daraus folgt, dass Knochen- oder Marknägel oder andere Implantate aus einer biodegradierbaren Magnesiumlegierung in einem menschlichen oder tierischen Körper eingesetzt werden können und sich dann, vorzugsweise erst nach Erfüllung einer medizinischen Funktion, ohne Weiteres auflösen. Dies ist ein entscheidender Vorteil gegenüber herkömmlichen Implantaten, da keine neuerliche Operation erforderlich ist, um ein Implantat nach Erfüllung einer medizinischen bzw. therapeutischen Funktion aus dem Körper zu entnehmen. Idealerweise sollte das Implantat so lange stabil bleiben, als eine medizinische bzw. therapeutische Funktion erwünscht ist und sich unmittelbar im Anschluss daran vollständig unter Einwirkung einer Körperflüssigkeit auflösen.

In diesem Zusammenhang werden insbesondere Magnesium-Zink-Legierungen angedacht, da Magnesium-Zink-Legierungen hohe Festigkeitswerte aufweisen, was für die Erfüllung einer medizinischen bzw. therapeutischen Funktion notwendig ist oder zumindest notwendig sein kann.

Um für bestimmte Anwendungen eine geforderte Mindestfestigkeit zu erreichen, werden auch in anderen Bereichen häufig Magnesium-Zink-Legierungen eingesetzt. Das Dokument WO 2009/148515 offenbart eine Magnesium-Zink-Legierung, die für Implantate vorgesehen ist. Mit derartigen Legierungen können insbesondere bei höheren Zinkgehalten zwar Körper hoher Festigkeit hergestellt werden, allerdings haben die Erfinder erkannt, dass oftmals eine hohe Warmrissneigung bei der Herstellung der Körper gegeben ist (sogenanntes "hot tearing"). Eine Warmrissneigung kann jedoch dazu führen, dass ein Gussbolzen im Oberflächenbereich, in Abhängigkeit einer geforderten Qualität, spanabhebend bearbeitet werden muss, um für eine weitere Verarbeitung, insbesondere eine Herstellung von Endprodukten, einwandfreies Material zu erhalten. Dies bedingt einen weiteren Verfahrensschritt, der nicht nur zusätzlichen Zeit- und Arbeitsaufwand mit sich bringt, sondern auch zu einem erheblichen Materialabfall führt. Eine Warmrissneigung kann aber auch so weit gehen, dass Heißrisse in der Mitte eines Gussstückes vorliegen, was zum Ausschuss desselben führt. Beispielsweise kann ein Bolzen nicht mehr verpresst werden, wenn in der Bolzenmitte ein Riss vorliegt.

Aufgabe der Erfindung ist es, eine Legierung der eingangs genannten Art anzugeben, bei welcher eine Warmrissneigung vermindert ist.

Ein weiteres Ziel der Erfindung ist es, eine Verwendung einer derartigen Legierung darzustellen.

Ferner ist es ein Ziel der Erfindung, ein Implantat anzugeben, das kosteneffektiv hergestellt werden kann.

Schließlich ist es ein Ziel der Erfindung, ein Verfahren der eingangs genannten Art anzugeben, mit welchem ein Körper aus einer Magnesiumlegierung ohne oder zumindest mit einer verringerten Anzahl von Warmrissen herstellbar ist.

Die zuvor genannte Aufgabe wird erfindungsgemäß durch eine Magnesiumlegierung gelöst, enthaltend (in Gewichtsprozent)
mehr als 0,0 bis zu 5,0 % Zink
optional mehr als 0,0 bis zu 1,0 % Zirconium
mehr als 0,0 bis zu 1,0 % Calcium
optional mehr als 0,0 bis zu 1,0 % Mangan
optional mehr als 0,0 bis zu 0,5 % Silicium
optional mehr als 0,0 bis zu 1,0 % Silber
maximal bis zu 0,5 % Aluminium
und zumindest ein Element ausgewählt aus der Gruppe bestehend aus
mehr als 0,05 bis zu 0,6 % Yttrium
mehr als 0,05 bis zu 1,2 % Ytterbium
mehr als 0,05 bis zu 1,2 % Gadolinium

Rest Magnesium und herstellungsbedingte Verunreinigungen.

Im Rahmen der Erfindung wurde erkannt, dass bei einer Magnesium-Zink-Legierung bereits niedrige Yttrium- und/oder Ytterbium- und/oder Gadoliniumgehalte zu einer deutlichen Reduktion einer Warmrissneigung der Magnesiumlegierung führen. Dies kann dadurch begründet werden, dass durch die Zugabe entsprechender Elemente bei einer Herstellung einer Schmelze intermetallische Phasen gebildet werden, wodurch eine Soliduslinie angehoben und ein Erstarrungsintervall verkleinert und damit eine für eine Warmrissneigung verantwortliche thermische Schrumpfung verringert wird. Gleichzeitig ergibt sich dadurch bei einer nachfolgenden thermischen Behandlung, insbesondere einer Extrusion, einem Strangpressen oder auch Walzen ein größeres thermisches Fenster, da die Magnesiumlegierung ohne partielles Aufschmelzen bei wesentlich höheren Temperaturen thermisch behandelt werden kann als bisher bekannte Legierungen dieses Typs.

Eine erfindungsgemäße Magnesiumlegierung enthält Zink, damit hohe mechanische Kennwerte erhalten werden, was für diverse Anwendungen wie Stents notwendig sein kann. Bevorzugt ist vorgesehen, dass die Magnesiumlegierung 1,0 bis 5,0 %, vorzugsweise 2,5 bis 4,5 %, Zink enthält. Eine erfindungsgemäße Legierung kann relativ hohe Gehalte an Zink von z. B. mehr als 2,5 % enthalten, was eine gewünscht hohe Festigkeit ergibt, ohne dass eine praktisch signifikante Warmrissneigung gegeben wäre.

Des Weiteren enthält eine erfindungsgemäße Magnesiumlegierung mehr als 0,0 bis zu 1,0 % Calcium und kann Zirconium bis zu 1,0 % enthalten, und zwar für die Zwecke einer Kornfeinung. Bevorzugt ist vorgesehen, dass die Magnesiumlegierung mehr als 0,1 bis 0,6 % Zirconium und/oder mehr als 0,1 bis 0,4 % Calcium enthält.

Zirconium und Calcium können grundsätzlich auch Bestandteile von intermetallischen Phasen sein, die bei der Herstellung der Magnesiumlegierung bzw. Abkühlung einer in der Zusammensetzung entsprechenden Schmelze gebildet werden. Um die Bildung gewünschter ternärer intermetallischer Phasen mit den Elementen Yttrium, Ytterbium und/oder Gadolinium auf der einen Seite nicht zu unterbinden, aber auf der anderen Seite auch eine gewünschte Kornfeinung sicherzustellen, beträgt ein Summengehalt von Zirconium und Calcium mit Vorteil 0,6 bis 1,0 %.

Des Weiteren kann eine erfindungsgemäße Magnesiumlegierung Mangan, Silicium und/oder Silber mit den zuvor genannten Maximalgehalten umfassen. Insbesondere kann vorgesehen sein, dass die Magnesiumlegierung zumindest ein Element ausgewählt aus der Gruppe bestehend aus
mehr als 0,1 bis zu 0,5 % Mangan
mehr als 0,1 bis zu 0,5 % Silicium
mehr als 0,1 bis zu 0,5 % Silber
enthält. Silber und Silicium tragen zu einer Feinkörnigkeit eines Gefüges der Magnesiumlegierung bei. Mangan verbessert eine Korrosionsbeständigkeit der Magnesiumlegierung.

Gehalte der Elemente Yttrium, Ytterbium und/oder Gadolinium sind bevorzugt so abgestimmt, dass die Magnesiumlegierung zumindest ein Element ausgewählt aus der Gruppe bestehend aus
mehr als 0,05 bis weniger als 0,5 % Yttrium
mehr als 0,1 bis zu 1,2 % Ytterbium
mehr als 0,1 bis zu 1,2 % Gadolinium
enthält. Dies ist dadurch begründet, dass Ytterbium und Gadolinium im Vergleich zu Yttrium etwa die doppelte Atommasse aufweisen. Sofern also die genannten Elemente einzeln vorliegen, ist relativ zu Yttrium eine doppelte Menge an Ytterbium oder Gadolinium vorzusehen, um analog wirkende intermetallische Phasen zu erhalten. Die genannten unteren Grenzen von 0,05 % für Yttrium bzw. 0,1 % für Ytterbium und Gadolinium stellen dabei Minimalgehalte dar, die in Bezug auf eine Bildung intermetallischer Phasen erforderlich sind. Bevorzugt ist es, dass eine untere Grenze auf 0,1 % für Yttrium und jeweils 0,2 % für Ytterbium und Gadolinium festgesetzt ist. Maximale Gehalte von 0,5 % für Yttrium und jeweils 1,2 % für Ytterbium und Gadolinium können zweckmäßig sein, um einerseits eine Warmrissneigung hintanzuhalten, andererseits aber auch eine Zytotoxizität von Implantaten möglichst gering zu halten. Insbesondere kann auch vorgesehen sein, dass mehrere der genannten Elemente gleichzeitig vorhanden sind, wobei ein maximaler Summengehalt weniger als 2,5 % beträgt.

Ein Aluminiumgehalt ist auf maximal bis zu 0,5 % begrenzt, weil Aluminium die vorteilhaften Wirkungen von Yttrium, Ytterbium und/oder Gadolinium maskieren kann.

Eine erfindungsgemäße Magnesiumlegierung kann in allen Bereichen eingesetzt werden, in welchen eine geringe Warmrissneigung gewünscht ist, beispielsweise bei einer Herstellung von Komponenten für Kraftfahrzeuge aus hochfesten Magnesiumlegierungen mit einem Zinkgehalt von mehr als 2,5 %. Bevorzugt ist jedoch vorgesehen, dass eine erfindungsgemäße Magnesiumlegierung zur Herstellung eines biodegradierbaren Implantats, insbesondere zum Einsatz in einem Markraum eines menschlichen Körpers, verwendet wird.

Das weitere Ziel der Erfindung wird erreicht, wenn ein Verfahren der eingangs genannten Art folgende Schritte umfasst:
a) Herstellung einer Schmelze, enthaltend
   mehr als 0,0 bis zu 5,0 % Zink
   optional mehr als 0,0 bis zu 1,0 % Zirconium
   mehr als 0,0 bis zu 1,0 % Calcium
   optional mehr als 0,0 bis zu 1,0 % Mangan
   optional mehr als 0,0 bis zu 0,5 % Silicium
   optional mehr als 0,0 bis zu 1,0 % Silber
   maximal bis zu 0,5 % Aluminium
   und zumindest ein Element ausgewählt aus der Gruppe bestehend aus
   mehr als 0,05 bis zu 0,6 % Yttrium
   mehr als 0,05 bis zu 1,2 % Ytterbium
   mehr als 0,05 bis zu 1,2 % Gadolinium
   Rest Magnesium und herstellungsbedingte Verunreinigungen,
b) Gießen der Schmelze zu einer festen Masse,
c) Glühen der festen Masse,
d) optional Umformen der festen Masse oder eines Teiles davon,
e) Herstellung des Körpers aus der optional umgeformten Masse oder Teilen davon.

Ein mit der Erfindung erzielter Vorteil ist darin zu sehen, dass ein Körper herstellbar ist, der eine geringe Warmrissneigung aufweist. Dadurch können aufwendige und zu unnötigem Abfall führende Bearbeitungsoperationen wie eine spanabhebende Bearbeitung einer Oberfläche des Körpers nach Herstellung desselben entfallen. Darüber hinaus eignet sich ein hergestellter Körper insbesondere zur weiteren Verarbeitung zu medizinischen Produkten, insbesondere Implantaten für die Osteosynthese, beispielsweise Knochenplatten, Knochenschrauben, Marknägel oder sogenannte Kirschner-Drähte. Dabei ist von Vorteil, dass sich die Magnesiumlegierung im Körper nach einer vorbestimmten Zeit aufgrund der Einwirkung von Körperflüssigkeiten von selbst auflöst. Das oder die vorgesehenen Elemente Yttrium, Ytterbium und/oder Gadolinium sorgen während der Herstellung dafür, dass ein Temperaturfenster für eine Schmelzeerstarrung relativ klein bzw. eine Soliduslinie im Vergleich mit einer Magnesium-Zink-Legierung angehoben ist, was zur gewünschten Verringerung einer Warmrissneigung auch bei Zinkgehalten über 2,5 % führt. Die Gehalte der einzelnen Elemente Yttrium, Ytterbium und/oder Gadolinium sind dabei vorteilhafterweise so abgestimmt, dass sich während einer Phase der Erstarrung der Schmelze aufgrund der Verteilungskoeffizienten intermetallische Phasen bilden, wobei es unter dem Einfluss der Seigerung zu einer Konzentrationssteigerung des oder der Elemente kommt, deren Aktivität bereits frühzeitig in einem Erstarrungsintervall zur Ausscheidung einer ternären intermetallischen Verbindung bzw. Verbindungen des oder der Elemente mit Magnesium und Zink unter Verwendung der gesamten Restschmelze führt, wodurch Zink für eine sonst erst bei wesentlich tieferen Temperaturen eintretende Reaktion mit Bildung einer binären metallischen Verbindung aus Magnesium und Zink nicht mehr oder nur eingeschränkt zur Verfügung steht, was zu einer Anhebung der Soliduslinie führt. Ferner sind Gehalte der Elemente Yttrium, Ytterbium und/oder Gadolinium so abgestimmt, dass sich bei Einsatz der Magnesiumlegierung als Implantat die intermetallischen Phasen und die Matrix etwa gleich schnell auflösen, sodass nach Auflösung des Implantats keine unerwünschten zytotoxischen Bestandteile im Körper vorhanden sind.

Als Gießverfahren kommt in der Regel ein Stranggießen zur Anwendung, wenngleich auch Niederdruckguss-, Druckguss-, Sand- und Kokillengussverfahren einsetzbar sind. Ein Glühen wird bevorzugt bei einer Glühtemperatur von 280 °C bis 400 °C durchgeführt. Sofern ein Umformen, z. B. ein Strangpressen, Equal Channel Angular Pressing (bekannt auch als "ECAP") und/oder Schmieden, vorgesehen ist, wird dieses unterhalb der Glühtemperatur durchgeführt.

Bei einem erfindungsgemäßen Verfahren kann es von Vorteil sein, die Gehalte einzelner Elemente wie vorstehend angeführt einzuschränken, um die ebenfalls bereits angeführten Vorteile zu erreichen.

Weitere Merkmale, Vorteile und Wirkungen der Erfindung ergeben sich aus den nachfolgend dargestellten Ausführungsbeispielen. In den Zeichnungen, auf welche dabei Bezug genommen wird, zeigen:
Fig. 1 ein Diagramm betreffend eine Warmrissneigung;
Fig. 2 ein Diagramm betreffend eine Warmrissneigung in Abhängigkeit eines Erstarrungsintervalls;
Fig. 3 einen isothermen Schnitt bei 300 °C für Magnesium-Zink-Legierungen mit verschiedenen Gehalten an Yttrium und Gadolinium.

Magnesium-Zink-Legierungen mit verschiedenen Gehalten an Yttrium, Ytterbium und/oder Gadolinium wurden bei 700 °C in eine Sternkokille aus einem Stahl abgegossen. Die sternförmigen Komponenten hatten längliche, stangenförmige Bereiche mit einer Länge von 25, 45, 65, 95, 125 und 175 mm; ein Durchmesser war konstant 10 mm. Für jede Magnesium-Zink-Legierung wurden fünf derartige Komponenten erstellt. Die Sternkokille aus Stahl wurde vor dem Abgießen auf eine Temperatur von 250 ± 5 °C erhitzt. Die Gießtemperatur von 700 °C entsprach einer Überhitzung der Schmelzen von etwa 60 °C. Nach dem Abgießen wurde die Sternkokille für fünf Minuten ruhen gelassen und anschließend geöffnet, um die abgegossenen Formteile zu untersuchen.

In der vorstehenden Weise wurden Magnesium-Zink-Legierungen mit Yttriumgehalten von bis zu 0,8 % hergestellt. Die Legierungen enthielten neben Magnesium 3,0 % Zink, etwa 0,4 % Zirconium, etwa 0,3 % Calcium sowie optional 0,15 % Mangan, 0,4 bis zu 0,8 % Yttrium und/oder 2,0 % Ytterbium und/oder 2,0 % Gadolinium.

Die einzelnen Komponenten wurden im Anschluss in Bezug auf Risse untersucht, wobei eine Bewertung der Güte der einzelnen Segmente anhand einer optischen Untersuchung der länglichen Bereiche der Komponenten erfolgte. Die Proben wurden des Weiteren auf Risse im Inneren untersucht.

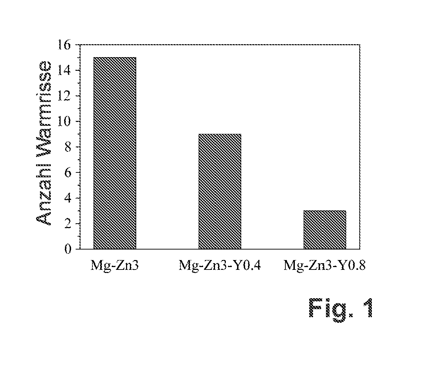

Im Inneren der einzelnen Komponenten konnten keine Risse festgestellt werden. In Bezug auf eine Anzahl der Risse an einer Oberfläche ergab sich für mit Yttrium hergestellte Magnesiumlegierungen, dass keine oder nur eine geringe Anzahl von Warmrissen vorhanden war. Mit zunehmendem Yttriumgehalt nahmen die Warmrisse ab. Ein ähnliches Bild ergab sich bei weiteren Versuchen, wobei entsprechende Schmelzen nicht in sternförmige Kokillen, sondern im sogenannten "direct chill casting" bzw. durch Stranggießen erstellt wurden; die entsprechenden Daten sind in Fig. 1 dargestellt. Aus Fig. 1 geht klar hervor, dass eine Warmrissneigung mit zunehmendem Yttriumgehalt abnimmt. Allerdings steigt mit zunehmendem Yttriumgehalt in einer Magnesiumlegierung auch das Risiko, dass sich ein Implantat aus der Magnesiumlegierung im Körper nach einer vorgegebenen Zeit auflöst, jedoch Yttrium zurückbleibt. Aus diesem Grund ist bei einer Verwendung entsprechender Magnesiumlegierungen für Implantate ein Yttriumgehalt auf maximal 0,6 %, bevorzugt weniger als 0,5 %, begrenzt.

Für Magnesiumlegierungen, bei welchen Yttrium durch Ytterbium ersetzt war, wurden ebenso wie für Magnesiumlegierungen mit Gadolinium entsprechende Ergebnisse erhalten. Mit anderen Worten: In allen Fällen konnte eine Warmrissneigung deutlich reduziert werden, wobei die Ytterbium- bzw. Gadoliniumgehalte doppelt so hoch sein konnten wie die Yttriumgehalte. Dies kann darauf zurückgeführt werden, dass sich intermetallische Phasen mit den drei genannten Elementen bilden, wobei aufgrund der Atommassen die Gehalte an Ytterbium und/oder Gadolinium deutlich höher sein können als jene von Yttrium.

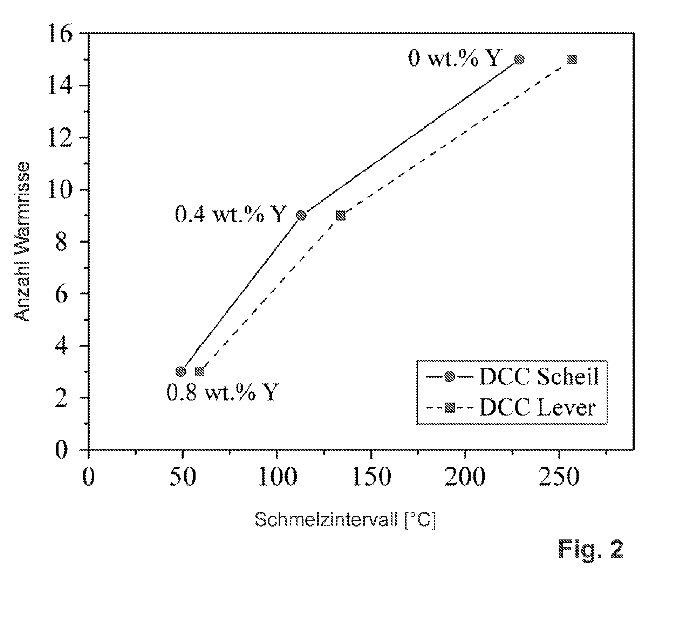

Durch ein Zulegieren von Yttrium, Ytterbium und/oder Gadolinium kann die Soliduslinie einer Magnesium-Zink-Legierung angehoben werden, was zu einer Reduktion der Warmrissneigung einer derartigen Magnesiumlegierung führt. Dies ist in Fig. 2 veranschaulicht. Wie ersichtlich, nimmt eine Anzahl von Warmrissen mit kleiner werdendem Schmelzintervall ab, wobei bereits bei Yttriumgehalten von weniger als 0,5 % günstige Ergebnisse erzielt werden.

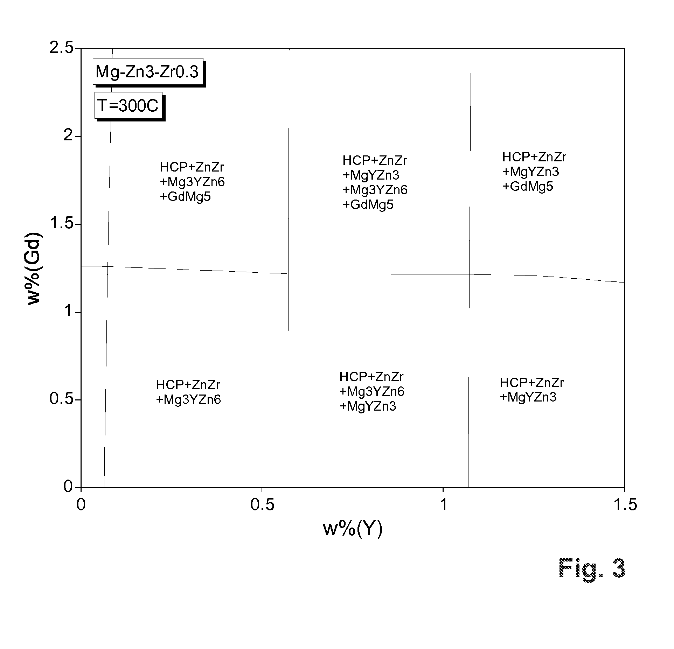

In Fig. 3 ist ein berechneter isothermer Schnitt bei 300 °C für Magnesium-Zink-Legierungen mit 3,0 % Zink, 0,3 % Zirconium und verschiedenen Gehalten an Yttrium und Gadolinium dargestellt. Die Temperatur von 300 °C entspricht einer typischen Extrusionstemperatur. Wie ersichtlich ist, ist bei Yttriumgehalten bis 0,6 % nur eine yttriumarme intermetallische Phase Mg₃YZn₆ vorhanden, während bei höheren Yttriumgehalten auch eine yttriumreiche Phase MgYZn₃ vorhanden ist. Die yttriumarme Phase wird für biodegradierbare Implantate als günstiger angesehen, wenn sich eine intermetallische Phase im Körper nicht oder nur sehr langsam auflösen sollte, da eine lokale Konzentration dann geringer ist. Darüber hinaus wird auch eine bessere Löslichkeit der yttriumarmen Phase vermutet.

Aus dem isothermen Schnitt gemäß Fig. 3 ergibt sich auch, dass Gadolinium zusätzlich zu Yttrium vorhanden sein kann, ohne dass sich an der Natur der Yttriumphasen etwas ändert. Insbesondere können auch ohne negative Wechselwirkung hohe Gadoliniumgehalte vorgesehen sein, wobei sich die Löslichkeit der intermetallischen Phasen nicht ändert.

Erfindungsgemäße Magnesiumlegierungen wurden wie erwähnt auch durch Stranggießen hergestellt, wobei eine durchschnittliche Korngröße des Gefüges wie auch im Kokillenguss für alle erfindungsgemäßen Magnesiumlegierungen kleiner als 50 µm war. Durch ein nachfolgendes Glühen bei einer Glühtemperatur von 280 °C bis 400 °C und ein anschließendes Strangpressen von Teilen der stranggegossenen Magnesiumlegierungen konnte eine weitere Reduktion der Korngröße erreicht werden. Insbesondere Magnesiumlegierungen mit Ytterbium, beispielsweise eine Magnesium-Zink-Legierung mit 2 % Ytterbium, zeigten sowohl hohe Festigkeiten als auch hohe Bruchdehnung (Dehngrenze R_{p0,2} etwa 300 MPa, Bruchdehnung A_{f} etwa 25 %). Magnesiumlegierungen mit derartig günstigen Eigenschaftswerten eignen sich nicht nur zur Herstellung von biodegradierbaren Implantaten, sondern können auch insbesondere für eine Herstellung von thermisch und/oder mechanisch stark beanspruchten Komponenten eingesetzt werden, beispielsweise Komponenten von Kraftfahrzeugen, die im Einsatz einer hohen statischen und/oder dynamischen Belastung unterliegen.

## Patentansprüche

1. Magnesiumlegierung, bestehend aus (in Gewichtsprozent)
mehr als 0,0 bis zu 5,0 % Zink
optional mehr als 0,0 bis zu 1,0 % Zirconium
mehr als 0,0 bis zu 1,0 % Calcium
optional mehr als 0,0 bis zu 1,0 % Mangan
optional mehr als 0,0 bis zu 0,5 % Silicium
optional mehr als 0,0 bis zu 1,0 % Silber
maximal bis zu 0,5 % Aluminium
und zumindest ein Element ausgewählt aus der Gruppe bestehend aus
mehr als 0,05 bis zu 0,6 % Yttrium
mehr als 0,05 bis zu 1,2 % Ytterbium
mehr als 0,05 bis zu 1,2 % Gadolinium
Rest Magnesium und herstellungsbedingte Verunreinigungen.

2. Magnesiumlegierung nach Anspruch 1, enthaltend 1,0 bis 5,0 %, vorzugsweise 2,5 bis 4,5 %, Zink.

3. Magnesiumlegierung nach Anspruch 1 oder 2, enthaltend mehr als 0,1 bis 0,6 % Zirconium und/oder mehr als 0,1 bis 0,4 % Calcium.

4. Magnesiumlegierung nach Anspruch 3, wobei ein Summengehalt von Zirconium und Calcium 0,6 bis 1,0 % beträgt.

5. Magnesiumlegierung nach einem der Ansprüche 1 bis 4, enthaltend zumindest ein Element ausgewählt aus der Gruppe bestehend aus
mehr als 0,1 bis zu 0,5 % Mangan
mehr als 0,1 bis zu 0,5 % Silicium
mehr als 0,1 bis zu 0,5 % Silber.

6. Magnesiumlegierung nach einem der Ansprüche 1 bis 5, enthaltend zumindest ein Element ausgewählt aus der Gruppe bestehend aus
mehr als 0,05 bis weniger als 0,5 % Yttrium
mehr als 0,1 bis zu 1,2 % Ytterbium
mehr als 0,1 bis zu 1,2 % Gadolinium.

7. Magnesiumlegierung nach einem der Ansprüche 1 bis 6, enthaltend zumindest zwei Elemente ausgewählt aus der Gruppe Yttrium, Ytterbium und Gadolinium, wobei ein Summengehalt dieser Elemente weniger als 2,5 % beträgt.

8. Verwendung einer Magnesiumlegierung nach einem der Ansprüche 1 bis 7 zur Herstellung eines biodegradierbaren Implantats, insbesondere zum Einsatz in einem Markraum eines menschlichen Körpers.

9. Implantat aus einer Magnesiumlegierung nach einem der Ansprüche 1 bis 7.

10. Verfahren zur Herstellung eines Körpers, insbesondere eines Implantats, aus einer Magnesiumlegierung, umfassend folgende Schritte:
a) Herstellung einer Schmelze, bestehend aus
mehr als 0,0 bis zu 5,0 % Zink
optional mehr als 0,0 bis zu 1,0 % Zirconium
mehr als 0,0 bis zu 1,0 % Calcium
optional mehr als 0,0 bis zu 1,0 % Mangan
optional mehr als 0,0 bis zu 0,5 % Silicium
optional mehr als 0,0 bis zu 1,0 % Silber
maximal bis zu 0,5 % Aluminium
und zumindest ein Element ausgewählt aus der Gruppe bestehend aus mehr als 0,05 bis zu 0,6 % Yttrium
mehr als 0,05 bis zu 1,2 % Ytterbium
mehr als 0,05 bis zu 1,2 % Gadolinium
Rest Magnesium und herstellungsbedingte Verunreinigungen,
b) Gießen der Schmelze zu einer festen Masse,
c) Glühen der festen Masse,
d) optional Umformen der festen Masse oder eines Teiles davon,
e) Herstellung des Körpers aus der optional umgeformten Masse oder Teilen davon.

11. Verfahren nach Anspruch 10, wobei das Glühen bei einer Glühtemperatur von 280 °C bis 400 °C durchgeführt wird.

12. Verfahren nach Anspruch 10 oder 11, wobei ein Umformen bei einer Temperatur unterhalb der Glühtemperatur durchgeführt wird.

13. Verfahren nach einem der Ansprüche 10 bis 12, wobei das Umformen durch Strangpressen, Equal Channel Angular Pressing und/oder Schmieden durchgeführt wird.

## Claims

1. A magnesium alloy consisting of (as percentage by weight)
more than 0.0 to 5.0 % zinc
optionally more than 0.0 to 1.0 % zirconium
more than 0.0 to 1.0 % calcium
optionally more than 0.0 to 1.0 % manganese
optionally more than 0.0 to 0.5 % silicon
optionally more than 0.0 to 1.0 % silver
up to 0.5 % aluminium as a maximum
and at least one element chosen from the group comprising
more than 0.05 % to 0.6 % yttrium
more than 0.05 to 1.2 % ytterbium
more than 0.05 to 1.2 % gadolinium
residual magnesium and production-related impurities.

2. The magnesium alloy according to claim 1, containing 1.0 to 5.0 %, preferably 2.5 to 4.5 %, zinc.

3. The magnesium alloy according to claim 1 or 2, containing more than 0.1 to 0.6 % zirconium and/or more than 0.1 to 0.4 % calcium.

4. The magnesium alloy according to claim 3, wherein a total content of zirconium and calcium amounts to 0.6 to 1.0 %.

5. The magnesium alloy according to one of claims 1 to 4, containing at least one element chosen from the group comprising
more than 0.1 to 0.5 % manganese
more than 0.1 to 0.5 % silicon
more than 0.1 to 0.5 % silver.

6. The magnesium alloy according to one of claims 1 to 5, containing at least one element chosen from the group comprising
more than 0.05 to less than 0.5 % yttrium
more than 0.1 to 1.2 % ytterbium
more than 0.1 to 1.2 % gadolinium.

7. The magnesium alloy according to one of claims 1 to 6, containing at least two elements chosen from the group comprising yttrium, ytterbium and gadolinium, wherein a total content of these elements amounts to less than 2.5 %.

8. Use of a magnesium alloy according to one of claims 1 to 7 for the production of a biodegradable implant, in particular for use in a medullary cavity of a human body.

9. An implant made of a magnesium alloy according to one of claims 1 to 7.

10. A method for the production of a body, in particular an implant, made of a magnesium alloy, comprising the following steps:
a) production of a melt consisting of
more than 0.0 to 5.0 % zinc
optionally more than 0.0 to 1.0 % zirconium
more than 0.0 to 1.0 % calcium
optionally more than 0.0 to 1.0 % manganese
optionally more than 0.0 to 0.5 % silicon
optionally more than 0.0 to 1.0 % silver
up to 0.5 % aluminium as a maximum and at least one element chosen from the group comprising
more than 0.05 to 0.6 % yttrium
more than 0.05 to 1.2 % ytterbium
more than 0.05 to 1.2 % gadolinium
residual magnesium and production-related impurities,
b) casting of the melt into a solid mass,
c) annealing of the solid mass,
d) optional reshaping of the solid mass or a part thereof,
e) production of the body from the optionally reshaped mass or parts thereof.

11. The method according to claim 10, wherein the annealing is carried out at an annealing temperature of 280 °C to 400 °C.

12. The method according to claim 10 or 11, wherein reshaping is carried out at a temperature below the annealing temperature.

13. The method according to one of claims 10 to 12, wherein reshaping is carried out by extrusion moulding, equal channel angular pressing and/or forging.

## Revendications

1. Alliage de magnésium, constitué de (en pourcentage en poids)
plus de 0,0 à 5,0 % de zinc,
en option, plus de 0,0 à 1,0 % de zirconium,
plus de 0,0 à 1,0 % de calcium,
en option, plus de 0,0 à 1,0 % de manganèse,
en option, plus de 0,0 à 0,5 % de silicium,
en option, plus de 0,0 à 1,0 % d'argent,
au maximum à 0,5 % d'aluminium
et d'au moins un élément choisi dans le groupe comprenant
plus de 0,05 à 0,6 % d'yttrium,
plus de 0,05 à 1,2 % d'ytterbium,
plus de 0,05 à 1,2 % de gadolinium
d'un reste de magnésium et d'impuretés dues à la production.

2. Alliage de magnésium selon la revendication 1, contenant de 1,0 à 5,0 %, de préférence de 2,5 à 4,5 % de zinc.

3. Alliage de magnésium selon la revendication 1 ou 2, contenant plus de 0,1 à 0,6 % de zirconium et/ou plus de 0,1 à 0,4 % de calcium.

4. Alliage de magnésium selon la revendication 3, une teneur totale de zirconium et de calcium s'élevant à de 0,6 à 1,0 %.

5. Alliage de magnésium selon l'une quelconque des revendications 1 à 4, contenant au moins un élément choisi dans le groupe comprenant
plus de 0,1 à 0,5 % de manganèse,
plus de 0,1 à 0,5 % de silicium,
plus de 0,1 à 0,5 % d'argent.

6. Alliage de magnésium selon l'une quelconque des revendications 1 à 5, contenant au moins un élément choisi dans le groupe comprenant
plus de 0,05 à moins de 0,5 % d'yttrium,
plus de 0,1 à 1,2 % d'ytterbium,
plus de 0,1 à 1,2 % de gadolinium.

7. Alliage de magnésium selon l'une quelconque des revendications 1 à 6, contenant au moins deux éléments choisis dans le groupe yttrium, ytterbium et gadolinium, une teneur totale desdits éléments s'élevant à moins de 2,5 %.

8. Utilisation d'un alliage de magnésium selon l'une quelconque des revendications 1 à 7 pour fabriquer un implant biodégradable, notamment destiné à l'utilisation dans un canal médullaire d'un corps humain.

9. Implant en un alliage d'aluminium selon l'une quelconque des revendications 1 à 7.

10. Procédé destiné à fabriquer un corps, notamment un implant en un alliage de magnésium, comprenant les étapes suivantes :
a) préparation d'une masse fondue, constituée de
plus de 0,0 à 5,0 % de zinc,
en option, plus de 0,0 à 1,0 % de zirconium,
plus de 0,0 à 1,0 % de calcium,
en option, plus de 0,0 à 1,0 % de manganèse,
en option, plus de 0,0 à 0,5 % de silicium,
en option, plus de 0,0 à 1,0 % d'argent,
au maximum à 0,5 % d'aluminium
et d'au moins un élément choisi dans le groupe comprenant
plus de 0,05 à 0,6 % d'yttrium,
plus de 0,05 à 1,2 % d'ytterbium,
plus de 0,05 à 1,2 % de gadolinium
d'un reste de magnésium et d'impuretés dues à la production,
b) coulée de la masse fondue en une masse solide,
c) calcination de la masse solide,
d) en option, façonnage de la masse solide ou d'une partie de celle-ci,
e) fabrication du corps à partir de la masse façonnée en option ou de parties de celle-ci.

11. Procédé selon la revendication 10, la calcination étant réalisée à une température de 280° C à 400°C.

12. Procédé selon la revendication 10 ou 11, un façonnage étant réalisé à une température inférieure à la température de calcination.

13. Procédé selon l'une quelconque des revendications 10 à 12, le façonnage étant réalisé par extrusion, par Equal Channel Angular Pressing (extrusion en canal angulaire) et/ou par forgeage.
